# EUROPÄISCHE PATENTANMELDUNG

(11) **EP 1 291 652 A2**
(43) Veröffentlichungstag der Anmeldung: **12.03.2003**
(21) Anmeldenummer: 02017802.6
(22) Anmeldetag: 07.08.2002
(51) Int. Cl.: G01N 33/02

(54) **Testgerät und Verfahren zur Erzeugung eines Testsignals für eine Lebensmittelverträglichkeitsanalyse**

(30) Priorität: 06.09.2001 DE 10143853; 13.09.2001 DE 10145249
(71) Anmelder: HoriSan GmbH, 46399 Bocholt (DE)
(72) Erfinder: Holzrichter, Klaus-Dieter, 46399 Bocholt (DE); Jossner, Dieter, 77866 Rheinau (DE)
(74) Vertreter: Gesthuysen, von Rohr & Eggert

(57) **Zusammenfassung**

Dargestellt und beschrieben sind ein Testgerät und ein Verfahren zur Erzeugung eines Testsignals für eine Lebensmittelverträglichkeitsanalyse mit einer Signalerzeugungseinrichtung (12), von der ein elektrisches Signal mit einem vorbestimmten Frequenzspektrum erzeugbar ist und die mit wenigstens einer eine Probesubstanz enthaltenden Probe (18) elektrisch koppelbar ist, und einem Ausgang (23), über den das von der Signalerzeugungseinrichtung (12) erzeugte Signal bei elektrischer Kopplung mit der Probe (18) ausgebbar ist. Erfindungsgemäß kann somit auf einfache, sichere und schnelle Art und Weise eine Lebensmittelverträglichkeitsanalyse durchgeführt werden.

## Beschreibung

Die Erfindung betrifft ein Testgerät und ein Verfahren zur Erzeugung eines Testsignals für eine Lebensmittelverträglichkeitsanalyse.

Der Zweck von Lebensmittelverträglichkeitsanalysen liegt darin, eine für eine einzelne Person individuelle Ernährung zu finden. Die Zusammensetzung der Nahrung in Bezug auf die Auswahl der einzelnen Nahrungsmittel und auf die prozentuale Zusammenstellung, nämlich den prozentualen Anteil an Kohlenhydraten, Eiweiß und Fett, muß sich an der Funktion des Stoffwechsels, also der Verarbeitung der zugeführten Nahrung zum Aufbau von Energie, orientieren. Wenn eine Person Nahrungsmittel zu sich nimmt, welche ihr Stoffwechsel gegenwärtig nicht verwerten kann, kommt es zu entsprechenden gesundheitlichen Störungen.

Basierend auf den Forschungen von Prof. Schole, nämlich seiner Dreikompententheorie, und auf den Beobachtungen verschiedener Autoren aus den USA stellt die Funktion des Stoffwechsels ein sehr labiles Gleichgewicht dar und ist abhängig von verschiedenen Faktoren. Dazu gehören einerseits die genetischen, nicht veränderbaren Faktoren, wie die Blutgruppenzugehörigkeit und die Zugehörigkeit zu einem der vier "Drüsentypen". Andererseits gehören dazu die veränderbaren Stoffwechseltypen, einerseits betreffend das autonome Nervensystem, entsprechend dem sympatischen Typ bzw. dem parasympatischen Typ, und andererseits betreffend das Verbrennungssystem, entsprechend dem Schnellverbrenner, dem Langsamverbrenner bzw. dem ausgeglichenen Typ.

Die zuvor genannten veränderbaren Stoffwechseltypen unterliegen einer Vielzahl von Einflüssen aus der Umwelt, wie Bakterien, Viren, Umweltgiften, Elektrostreß, geopathischen Belastungen, Medikamenten, falscher Auswahl von Nahrungsmitteln usw. Außerdem ist dabei die Psyche ein nicht zu vernachlässigender Faktor. Diese Vielzahl von Einflüssen führt dazu, daß das Stoffwechselgleichgewicht leicht aus dem Takt gerät. In einem solchen Fall entstehen auf der Stoffwechselebene für die betroffene Person nur schwer erkennbare Veränderungen', die dazu führen, daß nunmehr einzelne Nahrungsbestandteile, wie Kohlenhydrate, Eiweiße oder/und Fett, anders verarbeitet und vertragen werden als vorher. Infolge dessen stellen sich sogenannte Befindlichkeitsstörungen, wie Müdigkeit, Apathie, Gereiztheit, Schlaflosigkeit, Konzentrationsstörungen, aber auch Allergien, Depressionen und Gewichtsschwankungen, ein. Durch die unbewußte Fehlernährung kommt es zu merklichen Veränderungen in der Körperzusammensetzung mit den Folgen einer Mangelernährung und einer Veränderung im Säure-Basen-Haushalt. Dadurch entstehen weitere Veränderungen auf der hormonellen Ebene, welche einerseits das metabolische Ungleichgewicht weiter verstärken und andererseits zu Heißhungerattacken führen, durch die die betroffene Person ihre Eßverhalten ändert und typischerweise an Gewicht zunimmt.

Kommt eine solche Person in eine Ernährungsberatung, so wird ihr nach den heute geltenden Vorstellungen im allgemeinen empfohlen, sich von 60 % Kohlenhydraten, 25 % Eiweiß und 15 % Fett zu ernähren. Es werden jedoch auch andere Ernährungsformen empfohlen, wie die vegetarische Ernährung, Makrobiotik, Rohkost usw. All diese Empfehlungen für die Ernährung stellen für den einen oder anderen Stoffwechseltypen eine durchaus sinnvolle Ernährung dar, jedoch nicht für alle. Eine pauschale Ernährungsempfehlung, die sich in keiner Weise an der augenblicklichen Stoffwechselsituation des betroffenen Personen orientiert, beschert dem Betroffenen somit meist eine lange Odyssee von Diät zu Diät.

Es ist der Verdienst von Dr. Kelly und Dr. Wolcott, in den letzten dreißig Jahren das Wissen um die verschiedenen Stoffwechseltypen gemehrt zu haben. Den einzelnen Stoffwechseltypen können klare Empfehlungen bezüglich der Auswahl und Zusammensetzung der Nahrungsmittel zugeordnet werden. Ernährt sich eine Person ihrem aktuellen Stoffwechseltyp entsprechend, werden sich in kurzer Zeit deutliche Verbesserungen im Befinden einstellen, und es kommt zu laborchemischen Veränderungen im Sinne einer Verbesserung der Werte. Über- bzw. Untergewicht werden sich normalisieren, und die Person wird ihre Gewicht halten, solange sie sich an den Empfehlungen für den aktuellen Stoffwechseltyp orientiert.

Da sich der Stoffwechseltyp jedoch wieder ändern kann, ist es sinnvoll, den Stoffwechseltyp öfter zu kontrollieren. Bei gesunden Personen reicht im allgemeinen eine Kontrolle alle sechs bis zwölf Monate aus. Bei Personen mit gesundheitlichen Problemen oder Gewichtsproblemen sollten Kontrollen alle vier bis sechs Wochen erfolgen.

Dr. Kelly und Dr. Wolcott bestimmen den Stoffwechseltyp über eine Kombination von etwa 250 Multiple-Choice-Fragen zusammen mit sehr aufwendigen laborchemischen Untersuchungen. Der Zeitaufwand beträgt pro Patient mehrere Stunden bis zu Tagen, so daß die Bestimmung sehr kostenaufwendig ist. Insbesondere aus diesem Grund ist dieses System der Stoffwechseltypisierung noch nicht weiter verbreitet.

Zur Lebensmittelverträglichkeitsanalyse ist ferner ein kinesiologischer Muskeltest bekannt, bei dem der zu testenden Personen Lebensmittel in nach Möglichkeit neutralen Behältnissen auf die Brust gehalten werden, um ihre Reaktion darauf zu testen. Problematisch dabei ist jedoch, daß Störeinflüsse von außen nur schlecht abgeschirmt werden können und die Reaktion der zu testenden Person oft nur schwach ist.

Schließlich ist es zur Lebensmittelverträglichkeitsanalyse bekannt, der zu testenden Person die zu testenden Substanzen oral zu verabreichen, was jedoch insofern nachteilig ist, als daß dieses Verfahren viel Zeit in Anspruch nimmt und Wechselwirkungen mit anderen Lebensmitteln, die die zu testende Person zu sich genommen hat, nicht ausgeschlossen werden können.

Dementsprechend ist es die Aufgabe der Erfindung, ein Testgerät bzw. ein Verfahren bereitzustellen, mit denen auf einfache und sichere Weise in kurzer Zeit die individuelle und optimale Ernährung, die Zugehörigkeit zu einem Stoffwechseltypen, die Zugehörigkeit zu einem "Drüsentypen" und geeignete Nahrungsergänzungsprodukte für eine einzelnen Person bestimmt werden können, um letztlich die Ernährungssituation der Person zu verbessern.

Die zuvor hergeleitete und aufgezeigte Aufgabe ist erfindungsgemäß gelöst durch ein Testgerät zur Erzeugung eines Testsignals für eine Lebensmittelverträglichkeitsanalyse, mit einer Signalerzeugungseinrichtung, von der ein elektrisches Signal mit einem vorbestimmten Frequenzspektrum erzeugbar ist und die mit wenigstens einer eine Probesubstanz enthaltenden Probe elektrisch koppelbar ist, und mit einem Ausgang, über den das von der Signalerzeugungseinrichtung erzeugte Signal bei elektrischer Kopplung mit der Probe ausgebbar ist.

Das über den Ausgang des erfindungsgemäßen Testgeräts bei elektrischer Kopplung mit der Probe ausgegebene, ursprünglich von der Signalerzeugungseinrichtung erzeugte Signal stellt also das Testsignal dar. Das Testsignal ist damit ein solches Signal, auf das die eine Probesubstanz enthaltende Probe durch die elektrische Kopplung Einfluß nimmt. Wenn auch unter Umständen meßtechnisch nur schwer nachweisbar, so ist das von der Signalerzeugungseinrichtung ursprünglich erzeugte Signal durch die elektrische Kopplung mit der Probe zumindest insoweit beeinflußt bzw. verändert worden, daß dies anhand von körperlichen Reaktionen der getesteten Person erkennbar ist. Bei einer Trennung der elektrischen Kopplung zwischen der Probe und der Signalerzeugungseinrichtung bleibt die bei vorhandener elektrischer Kopplung bei den getesteten Personen erzeugte Reaktion nämlich aus. Dies zeigen umfängliche Studien der Anmelderin, im Rahmen derer eine Vielzahl von Personen mit dem erfindungsgemäßen Testgerät getestet worden sind.

Durch das erfindungsgemäße Testgerät wird somit eine Lebensmittelverträglichkeitsanalyse ermöglicht, die auf den Forschungsergebnissen von Prof. Schole und Prof. Lutz ("Dreikomponententheorie" von 1982) beruht. Prof. Schole konnte zeigen, daß der menschliche Organismus auf von außen kommende Testsignale anspricht. Dieses Prinzip wird von dem erfindungsgemäßen Testgerät ausgenutzt: In Abhängigkeit von der Art der Probesubstanz kommt es im Körper zu mehr oder weniger starken Reaktionen, die z. B. mit Methoden der Naturheilkunde, wie Kinesiologie, Physioenergetik, RAC (Pulstastung), Biotensor oder EAV, an der getesteten Person über eine Veränderung des autonomen Nervensystems nachgewiesen werden können.

Es hat sich gezeigt, daß mit dem erfindungsgemäßen Testgerät beste Ergebnisse erzielt werden, wenn das von der Signalerzeugungseinrichtung erzeugte Signal ein breitbandiges Rauschsignal ist. Im allgemeinen ist es dabei ausreichend, daß sich der Frequenzbereich von einigen Hertz bis ca. 600 kHz erstreckt. Gemäß einer bevorzugten Weiterbildung der Erfindung ist jedoch vorgesehen, daß sich der Frequenzbereich von ca. 0,5 Hz bis 100 MHz erstreckt. Auf diese Weise wird sichergestellt, daß praktisch jede erdenkliche Probesubstanz als Probe verwendet werden kann.

Grundsätzlich ist es möglich, als Amplitude für das von der Signalerzeugungseinrichtung erzeugte Signal all solche Spannungen zu wählen, die für die zu testende Person gesundheitlich unbedenklich sind. Gemäß einer bevorzugten Weiterbildung einer Erfindung ist jedoch vorgesehen, daß das von der Signalerzeugungseinrichtung erzeugte Signal eine maximale Amplitude von unter 1 V aufweist, wobei die maximale Amplitude vorzugsweise im Bereich von 200 bis 300 mV liegt.

Grundsätzlich ist es auch möglich, daß das von der Signalerzeugungseinrichtung erzeugte Signal als kontinuierliches Signal der zu testenden Person zugeführt wird. Gemäß einer bevorzugten Weiterbildung der Erfindung ist jedoch vorgesehen, daß das über den Ausgang ausgebbare Signal mit einer Pulseinrichtung pulsbar ist. Dabei hat es sich als besonders vorteilhaft herausgestellt, wenn die Pulsfrequenz einer biologisch neutralen Frequenz entspricht und dabei vorzugsweise im Bereich zwischen 1900 und 2500 Hz und ganz besonders bevorzugt bei ca. 2000 Hz liegt.

Es gibt verschiedene Möglichkeiten, das über den Ausgang des Testgeräts ausgegebene Signal der zu testenden Person zuzuführen. Vorzugsweise erfolgt die Zuführung jedoch über eine an dem Ausgang des Testgeräts angeschlossene elektrische Leitung, die in einem um ein Handgelenk der zu testenden Person legbaren Armband endet. Dabei ist vorgesehen, daß die Haut der zu testenden Person über das elektrisch leitfähige Armband in direkte Kontakt mit dem Testsignal kommt.

Eine Verwendungsmöglichkeit des von dem Testgerät erzeugten Testsignals für eine Lebensmittelverträglichkeitsanalyse besteht darin, zu prüfen, ob bei einer bestimmten Probesubstanz eine Reaktion des getesteten Personen auftritt. Um nun jedoch auch eine quantitative Aussage über die Reaktion der getesteten Person auf eine bestimmte Probesubstanz erhalten zu können, ist gemäß einer bevorzugten Weiterbildung der Erfindung vorgesehen, daß die Leistung des über den Ausgäng ausgegebenen Signals steuerbar ist. Vorzugsweise ist dafür vorgesehen, daß das Testgerät über ein Drehrad verfügt, über das die Leistung des Signals vorbestimmt werden kann. Dabei kann die Steuerung der Leistung des über den Ausgang ausgegebenen Signals mit Hilfe von unterschiedlichen Verfahren erfolgen. So ist es z. B. möglich, die Leistung des über den Ausgang ausgegebenen Signals mittels der Amplitude des Signals zu steuern. Gemäß einer bevorzugten Weiterbildung der Erfindung ist jedoch vorgesehen, daß die Steuerung der Leistung des über den Ausgang ausgegebenen Signals über eine Pulsbreitmodulation erfolgt. Außerdem ist für die Leistung des über den Ausgang ausgegebenen Signals vorzugsweise eine Anzeigeeinrichtung vorgesehen, wobei sich eine Anzeigeeirnichtung in Form einer Prozentanzeige, vorzugsweise von 0 bis 100 % in 5 %-Schritten, besonders bewährt hat.

Auch bezüglich der elektrischen Kopplung der Probe mit der Signalerzeugungseinrichtung sind grundsätzlich unterschiedliche Vorgehensweisen möglich. Gemäß einer bevorzugten Weiterbildung der Erfindung ist diesbezüglich jedoch vorgesehen, daß die elektrische Kopplung der Probe mit der Signalerzeugungseinrichtung über einen Dual-Gate MOSFET erfolgt. Eine solche elektrische Kopplung ist insbesondere vorteilhaft, wenn die Steuerung der Leistung des über den Ausgang ausgegebenen Signals mittels einer Pulsbreitenmodulation erfolgt. Darüber hinaus, jedoch auch alternativ dazu, ist gemäß einer bevorzugten Weiterbildung der Erfindung vorgesehen, daß zwischen die Signalerzeugungseinrichtung und die Probe, und, falls vorgesehen, vor den Dual-Gate MOSFET, ein Verstärker geschaltet ist. Um die Verwendung der einzusetzenden Probesubstanzen möglichst nicht zu beschränken, ist dabei gemäß einer bevorzugten Weiterbildung der Erfindung vorgesehen, daß der Verstärker sehr breitbandig ist.

Obwohl das Testgerät grundsätzlich auch mit Wechselstrom betreibbar ist, ist gemäß einer bevorzugten Weiterbildung der Erfindung vorgesehen, daß das Testgerät mit Gleichstrom betrieben wird. Dies ist insbesondere insofern vorteilhaft, als daß dadurch keine Störfrequenzen erzeugt werden, die sich dem über den Ausgang des Testgeräts ausgegebenen Testsignal überlagern können. Zur Erzeugung des Gleichstroms ist, insbesondere beim Vorsehen der Möglichkeit des Anschließens des erfindungsgemäßen Testgeräts an eine standardgemäße 50 Hz-Netzwechselspannung, ein Netzteil vorgesehen, daß gemäß einer bevorzugten Weiterbildung der Erfindung außerhalb des Testgeräts angeordnet ist. Auf diese Weise wird auch eine Einkopplung der 50 Hz-Netzwechselspannung in das Testgerät und damit eine Überlagerung der 50 Hz-Netzwechselspannung mit dem über den Ausgang des Testgeräts ausgegebenen Testsignal vermieden. Darüber hinaus ist gemäß einer bevorzugten Weiterbildung der Erfindung vorgesehen, daß das Netzteil ein Filterelement und/oder ein Siebelement aufweist, was Störschwingungen in dem erfindungsgemäßen Testgerät weiter verringert.

Die Probe kann als Probesubstanz typischerweise jede Substanz enthalten, auf die die zu testende Person bezüglich einer Lebensmittelverträglichkeit analysiert werden soll. Insbesondere kommen dabei als Probesubstanzen Kohlenhydrate, Eiweiße, Fette, Enzyme usw. in Betracht. Dabei können die Probesubstanzen in der Probe unverdünnt enthalten sein. Gemäß ein bevorzugten Weiterbildung der Erfindung enthält die Probe jedoch die Probesubstanz in einer hohen, vorzugsweise in einer homöopathischen, Verdünnung. Dabei dient als Verdünnungsmittel vorzugsweise mehrfach destilliertes Wasser. Insbesondere kann die Probe auch von einer Nosode gebildet werden.

Um die Testmöglichkeiten mit dem erfindungsgemäßen Testgerät möglichst breit zu gestalten, wird gemäß einer bevorzugten Weiterbildung der Erfindung eine Mehrzahl von Proben mit voneinander verschiedenen Probesubstanzen bereitgestellt. Grundsätzlich ist es dabei möglich, die Proben außerhalb des Testgeräts vorzusehen und diese zum Testen z. B. einzeln in das erfindungsgemäße Testgerät einzusetzen. Gemäß einer bevorzugten Weiterbildung der Erfindung sind die Proben jedoch vollständig im Testgerät vorgesehen. Dann ist es möglich, für die Probe, die mit der Signalerzeugungseinrichtung elektrisch gekoppelt werden soll, die also an der zu testenden Person getestet werden soll, am Testgerät eine Vorwählbarkeit vorzusehen. In diesem Zusammenhang ist es vorteilhaft, für die vorgewählte Probe eine Anzeige vorzusehen, so daß immer erkennbar ist, welche Probe gegenwärtig getestet wird.

Beim Vorsehen einer Mehrzahl von Proben mit voneinander verschiedenen Probensubstanzen ist gemäß einer bevorzugten Weiterbildung der Erfindung vorgesehen, daß gleichzeitig mehr als eine dieser Proben in elektrische Kopplung mit der Signalerzeugungseinrichtung gebracht werden können. Auf diese Weise lassen sich komplexe Testsignale erzeugen, die die Testmöglichkeit des erfindungsgemäßen Testgeräts erweitern, insbesondere im Hinblick auf die Feststellung der Zugehörigkeit der getesteten Person zu den Stoffwechseltypen bzw. zu den "Drüsentypen". Die gleichzeitige elektrische Kopplung von mehreren Proben mit der Signalerzeugungseinrichtung ist ferner hilfreich zur Bestimmung geeigneter Nahrungsergänzungsprodukte, die der Verbesserung der Ernährungssituation dienen sollen.

Es hat sich als vorteilhaft herausgestellt, die Probesubstanzen in Ampullen abzufüllen, wobei die Ampullen vorzugsweise aus einem Metall, ganz besonders bevorzugt aus einem Edelmetall, wie Gold, hergestellt sind. Kostengünstige Ampullen mit hinreichend guten Eigenschaften bestehen z. B. aus Aluminium. Grundsätzlich ist jedoch aus Glas verwendbar, wobei in diesem Fall entsprechende Einrichtungen zur elektrischen Kopplung der in die Ampulle eingefüllten Probesubstanz mit der Signalerzeugungseinrichtung vorgesehen sein müssen. Dies kann z. B. ein elektrischer Leiter sein, der als Durchführung dient und in eine Ampulle aus Glas eingeschmolzen ist. Bei der Anordnung der Ampullen in dem erfindungsgemäßen Testgerät ist vorzugsweise ein Ampullenträger aus einem elektrische isolierendem Material vorgesehen. Dabei hat sich als elektrisch isolierendes Material für den Ampullenträger insbesondere Teflon besonders bewährt.

Die weiter oben hergeleitete und aufgezeigte Aufgabe ist ferner erfindungsgemäß gelöst durch ein Verfahren zur Erzeugung eines Testsignals für eine Lebensmittelverträglichkeitsanalyse, wobei von einer Signalerzeugungseinrichtung ein elektrisches Signal mit einem vorbestimmten Frequenzspektrum erzeugt wird, die Signalerzeugungseinrichtung mit wenigstens einer eine Probesubstanz enthaltenden Probe elektrisch gekoppelt wird und das von der Signalerzeugungseinrichtung erzeugte Signal bei elektrischer Kopplung mit der Probe ausgegeben wird.

Bevorzugte Weiterbildungen des erfindungsgemäßen Verfahrens ergeben sich in Analogie zu den zuvor beschriebenen bevorzugten Weiterbildungen des erfindungsgemäßen Testgeräts.

Im einzelnen gibt es nun eine Vielzahl von Möglichkeiten, das erfindungsgemäße Testgerät bzw. das erfindungsgemäße Verfahren auszugestalten und weiterzubilden. Dazu wird auf die den unabhängigen Patentansprüchen nachgeordneten Patentansprüche sowie auf die nachfolgende detaillierte Beschreibung eines bevorzugten Ausführungsbeispiels unter Bezugnahme auf die Zeichnung verwiesen. In der Zeichnung zeigt
- Fig. 1: ein Testgerät gemäß einem bevorzugten Ausführungsbeispiel der Erfindung,
- Fig. 2: schematisch den Gebrauch des Testgeräts gemäß dem bevorzugten Ausführungsbeispiel der Erfindung und
- Fig. 3: schematisch den Aufbau des Testgeräts gemäß dem bevorzugten Ausführungsbeispiel der Erfindung.

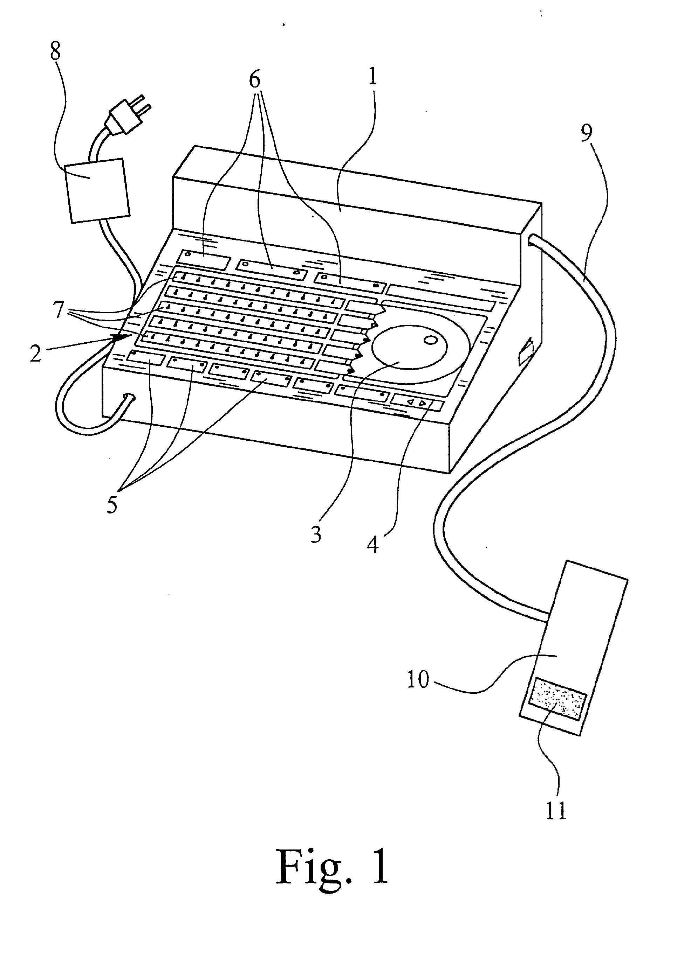

Aus Fig. 1 ist ein Testgerät zur Erzeugung eines Testsignals für eine Lebensmittelverträglichkeitsanalyse gemäß einem bevorzugten Ausführungsbeispiel der Erfindung ersichtlich. Das Testgerät weist ein Gehäuse 1 auf, das mit einer Bedien- und Anzeigefläche 2 versehen ist. Auf der Bedien- und Anzeigefläche 2 ist ein Drehrad 3 zur Einstellung der Leistung des Testsignals vorgesehen. In dem Gerät vorgesehene und daher in Fig. 1 nicht ersichtliche Proben lassen sich einzeln oder in Kombination über einen Wählschalter 4 voreinstellen, also in elektrische Kopplung mit der aus Fig. 1 ebenfalls nicht ersichtliche Signalerzeugungseinrichtung bringen, wobei dies über Anzeigen 5, 6 angezeigt wird. Je nach gewählter Probe bzw. je nach gewähltem Probenensemble wird die Leistung des Testsignals über eine Prozentanzeige 7 angezeigt. Bei einer alternativen Ausgestaltung des Testgeräts weisen die Anzeigen 5, 6 selbst Wählschalter für die einzelnen Proben auf.

Die Stromversorgung des Testgeräts erfolgt über ein Netzteil 8, das außerhalb des Gehäuses 1 des Testgeräts vorgesehen ist und einen nicht weiter dargestellten Filter sowie ein nicht weiter dargestelltes Siebelement aufweist. Auf diese Weise, also mit Hilfe des getrennt von dem Gehäuse 1 angeordneten Netzteils 8 sowie dem Vorsehen eines Filters sowie eines Siebelements in dem Netzteil 8, werden Störspannungen, insbesondere Störschwingungen, von dem Testgerät ferngehalten, so daß das Testsignal nicht negativ beeinflußt wird.

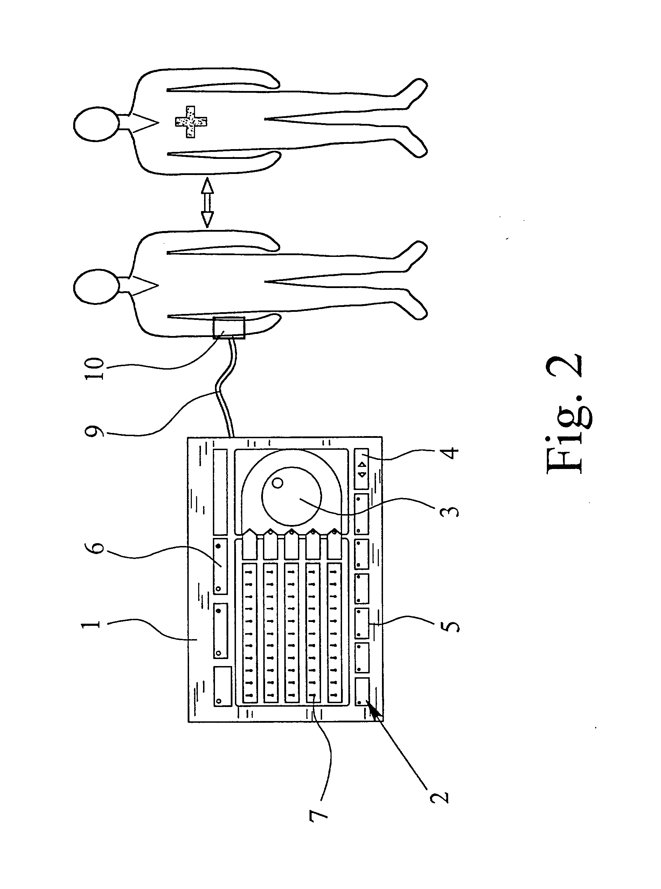

Die Zuführung des Testsignals zu der zu testenden Person erfolgt über eine elektrische Leitung 9, die in einem elektrisch leitenden Armband 10 endet, daß mit Hilfe eines Klettverschlusses 11 an einem Handgelenk der zu testenden Person befestigt werden kann. Dies ist schematisch aus Fig. 2 ersichtlich, in der der Einsatz des Testgeräts gemäß dem bevorzugten Ausführungsbeispiel der Erfindung schematisch dargestellt ist.

Wie zuvor schon angesprochen, wird durch das erfindungsgemäße Testgerät ein Testsignal für eine Lebensmittelverträglichkeitsanalyse bereitgestellt. Die Reaktion der mit diesem Testsignal getesteten Person erfolgt jedoch nicht über das erfindungsgemäße Testgerät, sondern über Verfahren, wie Kinesiologie, Physioenergetik, RAC, Biotensor oder EAV. Dieses Erfassen der körperlichen Reaktionen der getesteten Person durch einen den Test durchführenden Arzt oder Heilpraktiker ist schematisch durch den Doppelpfeil zwischen der zu testenden Person und dem Arzt bzw. Heilpraktiker angedeutet.

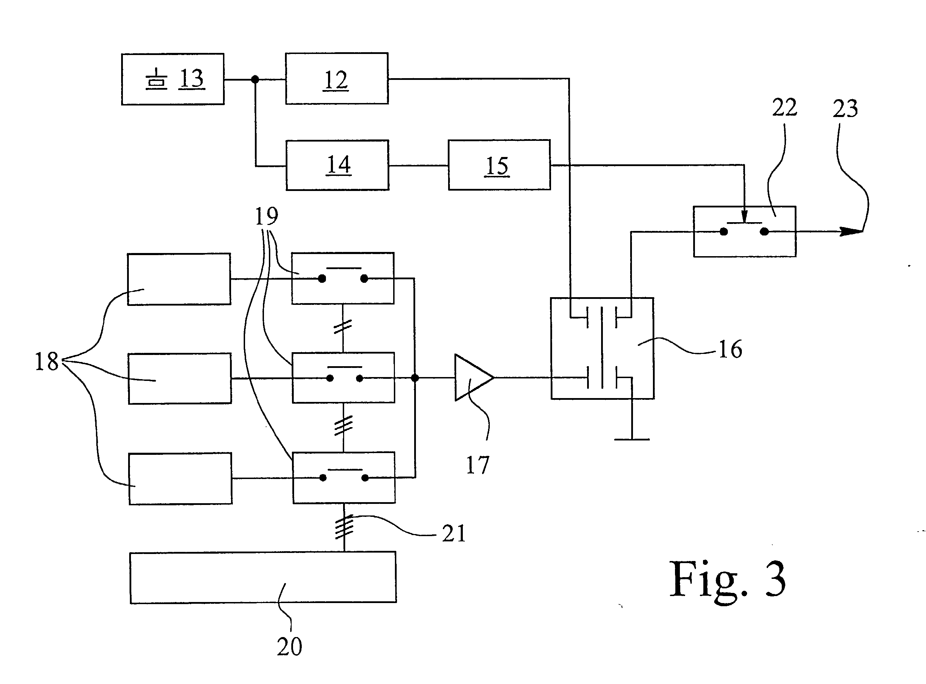

Aus Fig. 3 ist schließlich schematisch der Aufbau des erfindungsgemäßen Testgeräts ersichtlich. Als Signalerzeugungseinrichtung 12 dient eine Rauschgenerator, der an einen Quarzoszillator 13 angeschlossen ist. Der Quarzoszillator 13 ist ferner mit einem Pulsgenerator 14 verbunden. Dieser Pulsgenerator 14 erzeugt eine Impulsfolge mit einer Frequenz von ca. 2000 Hz. Auf den Pulsgenerator 14 folgt eine Pulsweiteneinstelleinrichtung 15, so daß schließlich ein pulsweitenmoduliertes Signal mit einer Frequenz von ca. 2000 Hz ausgegeben wird. Dieses Signal dient zum Pulsen des an die zu testende Person angelegten Signals, wie weiter unten im einzelnen erläutert.

Das von dem Rauschgenerator 12 ausgegebene Signal wird auf einen Dual-Gate MOSFET 16 geführt. An den Dual-Gate MOSFET 16 sind ferner über einen breitbandigen Verstärker 17 eine oder mehrere Proben 18 anschließbar. Der Anschluß der Proben erfolgt über elektronische Schalter 19, die über einen Vorwahlschalter 20 mittels einer Busleitung 21 angesteuert werden. Auf diese Weise werden eine oder mehrere angeschlossene Proben 18 über den Dual-Gate MOSFET 16 mit dem Rauschgenerator 12 elektrisch gekoppelt.

Das von dem Dual-Gaste MOSFET 16 ausgegebene Signal, das sowohl die Informationen des Rauschgenerators als auch die Informationen bezüglich der angeschlossenen Proben enthält, wird auf eine Signalpulseinrichtung 22 geführt, die auch das von der Pulsweiteneinstelleinrichtung 15 kommende Signal erhält. Auf diese Weise ist die Erzeugung eines pulsweitenmodulierten Testsignals möglich, das bei elektrischer Kopplung mit wenigstens einer Probe 18 Informationen über das von dem Rauschgenerator 12 kommende Signal enthält. Nicht dargestellt ist in Fig. 3, daß die Pulsweitmodulation extern steuerbar ist, so daß durch die Pulsweitmodulation die Leistung des Testsignals einstellbar ist. Die Signalpulseinrichtung 22 führt schließlich auf einen Ausgang 23 des Testgeräts, über den der Kontakt mit der zu testenden Person hergestellt wird.

Weiter oben ist schon angedeutet worden, daß der Fachmann bei dem Aufbau des Testgeräts gemäß dem bevorzugten Ausführungsbeispiel der Erfindung unter Umständen durch die elektrisch angekoppelten Proben 18 keine Beeinflussung des von dem Rauschgenerator 12 kommenden breitbandigen Signals erwarten und unter Umständen auch nur schwer meßtechnisch nachweisen können wird. Jedoch zeigen vielfältige Versuche, daß die bei einer getesteten Person erzeugbare Reaktion ganz wesentlich davon abhängt, ob eine Probe 18 an den Rauschgenerator 12 elektrisch angekoppelt ist oder nicht, und insbesondere auch, um was für eine Probe 18 es sich dabei handelt, also welche Probesubstanz sie enthält.

Der Vollständigkeit halber sei darauf hingewiesen, daß bei dem Testgerät gemäß dem bevorzugten Ausführungsbeispiel der Erfindung, wie in Fig. 3 nicht weiter gezeigt, die Proben 18 in Form von Aluminiumröhrchen vorliegen, in denen sich Probesubstanzen in homöopathischer Verdünnung befinden, wobei als Verdünnungsmittel dreifach destilliertes Wasser verwendet wird. Diese Aluminiumröhrchen sind in einem ebenfalls nicht weiter dargestellten Probenhalter angeordnet, der von einem Teflonblock mit entsprechenden Bohrungen gebildet wird.

## Patentansprüche

1. Testgerät zur Erzeugung eines Testsignals für eine Lebensmittelverträglichkeitsanalyse, mit einer Signalerzeugungseinrichtung (12), von der ein elektrisches Signal mit einem vorbestimmten Frequenzspektrum erzeugbar ist und die mit wenigstens einer eine Probesubstanz enthaltenden Probe (18) elektrisch koppelbar ist, und mit einem Ausgang (23), über den das von der Signalerzeugungseinrichtung (12) erzeugte Signal bei elektrischer Kopplung mit der Probe ausgebbar ist.

2. Testgerät nach Anspruch 1, **dadurch gekennzeichnet, daß** das von der Signalerzeugungseinrichtung (12) erzeugte Signal ein breitbandiges Rauschsignal ist, das vorzugsweise den Frequenzbereich von 0,5 Hz bis 100 MHz umfaßt.

3. Testgerät nach Anspruch 1 oder 2, **dadurch gekennzeichnet, daß** ein Pulsgenerator (14) vorgesehen ist, mit dem das über den Ausgang (23) ausgebbare Signal pulsbar ist.

4. Testgerät nach Anspruch 3, **dadurch gekennzeichnet, daß** die Pulsfrequenz des Pulsgenerators (14) einer biologisch neutralen Frequenz entspricht, vorzugsweise im Bereich zwischen 1900 und 2050 Hz und ganz besonders bevorzugt bei ca. 2000 Hz liegt.

5. Testgerät nach einem der Ansprüche 1 bis 4, **dadurch gekennzeichnet, daß** die Leistung des über den Ausgang (23) ausgegebenen Signals steuerbar ist, wobei die Steuerung der Leistung des über den Ausgang (23) ausgegebenen Signals vorzugsweise über eine Pulsbreitmodulation erfolgt.

6. Testgerät nach einem Ansprüche 1 bis 5, **dadurch gekennzeichnet, daß** die elektrische Kopplung der Probe (18) mit der Signalerzeugungseinrichtung (12) über einen Dual-Gate MOSFET (16) erfolgt.

7. Testgerät nach einem Ansprüche 1 bis 6 **dadurch gekennzeichnet, daß** zwischen die Signalerzeugungseinrichtung (12) und die Probe (18) ein vorzugsweise breitbandiger Verstärker (17) geschaltet ist.

8. Testgerät nach einem der Ansprüche 1 bis 7, **dadurch gekennzeichnet, daß** das Testgerät mit Gleichstrom betreibbar ist und zur Erzeugung des Gleichstroms ein außerhalb des Testgeräts angeordnetes Netzteil (8) vorgesehen ist.

9. Verfahren zur Erzeugung eines Testsignals für eine Lebensmittelverträglichkeitsanalyse, wobei von einer Signalerzeugungseinrichtung (12) ein elektrisches Signal mit einem vorbestimmten Frequenzspektrum erzeugt wird, die Signalerzeugungseinrichtung (12) mit wenigstens einer eine Probesubstanz enthaltenden Probe (18) elektrisch gekoppelt wird und das von der Signalerzeugungseinrichtung (12) erzeugte Signal bei elektrischer Kopplung mit der Probe (18) ausgegeben wird.

10. Verfahren nach Anspruch 9, **dadurch gekennzeichnet, daß** das von Signalerzeugungseinrichtung (12) erzeugte Signal ein breitbandiges Rauschsignal ist, das vorzugsweise den Frequenzbereich von 0,5 Hz bis 100 MHz umfaßt.

11. Verfahren nach Anspruch 9 oder 10, **dadurch gekennzeichnet, daß** das ausgegebene Signal gepulst wird.

12. Verfahren nach Anspruch 11, **dadurch gekennzeichnet, daß** die Pulsfrequenz einer biologisch neutralen Frequenz entspricht, nämlich vorzugsweise im Bereich zwischen 1900 und 2050 Hz und ganz besonders bevorzugt bei ca. 2000 Hz liegt.
